# EUROPEAN PATENT SPECIFICATION

(11) **EP 3 094 238 B1**
(45) Date of publication and mention of the grant of the patent: **02.10.2024**
(21) Application number: 15735041.4
(22) Date of filing: 13.01.2015
(51) Int. Cl.: A61M 16/00, A61B 5/08, A61B 5/087

(54) **SYSTEMS FOR DETERMINING EFFECTIVENESS OF RESPIRATION IN INDIVIDUALS**
SYSTEME ZUR BESTIMMUNG DER WIRKSAMKEIT VON BEATMUNG BEI PERSONEN
SYSTÈMES PERMETTANT DE DÉTERMINER UNE RESPIRATION EFFICACE CHEZ DES INDIVIDUS

(30) Priority: 13.01.2014 US 201461926434 P; 11.10.2014 US 201414512425
(43) Date of publication of application: 23.11.2016
(73) Proprietor: Xhale Assurance, Inc., Gainesville, FL 32608 (US)
(72) Inventor: MELKER, Richard, Newberry, FL 32669 (US); COHEN, Sean, Gainesville, FL 32605 (US); TAN, Huwei, Woburn, MA 01801 (US)
(74) Representative: Philips Intellectual Property & Standards
(86) International application number: PCT/US2015/011235
(87) International publication number: WO 2015/106280

(56) References cited:
- US-A1- 2007 027 375
- US-A1- 2008 302 364
- US-A1- 2010 016 694
- US-A1- 2013 006 075
- US-A1- 2013 133 655
- US-A1- 2014 005 557
- US-A1- 2014 128 697
- US-B1- 7 177 686

## Description

This application claims the benefit of U.S. Provisional Application No. 61/926,434, filed January 13, 2014, and this application is a continuation-in-part of U.S. Patent Application No. 14/512,425, filed on October 11, 2014, which claims priority to U.S. Provisional Patent Application 61/889,582, filed October 11, 2013.

### FIELD OF THE INVENTION

The present invention relates to biological sensors, and in particular, to photoplethysmography sensors. The present invention also relates to mammalian respiration and ventilation and devices for monitoring the same.

### BACKGROUND OF THE INVENTION

US 2010/016694 A1 discloses an air delivery system including a controllable flow generator operable to generate a supply of pressurized breathable gas to be provided to a patient for treatment and a pulse oximeter. The pulse oximeter may be configured to determine a measure of patient effort during a treatment period and provide a patient effort signal for input to control operation of the flow generator.

US 2013/133655 A1 discloses a method of providing high frequency ventilation to a patient, comprises delivering a flow of breathing gas to the patient, the flow of breathing gas having a first positive pressure level and a second positive pressure level, the first and second positive pressure levels alternating with one another in a plurality of cycles in the flow of breathing gas to have a frequency and an amplitude, the flow of breathing gas to the patient generating a mean airway pressure; determining whether the patient is breathing spontaneously or is trying to breath spontaneously; and, in response to the determination that the patient is breathing spontaneously or trying to breath spontaneously or according to the user settings for HFV and user intervention for non-spontaneous breathing patient, adjusting the mean airway pressure, modulating the frequency and duty cycle of the flow of breathing gas, or modulating the level of flow and pressure amplitude of the breathing gas.

US 2014/005557 A1 discloses photoplethysmography sensors including a clip body that includes a first end portion and a second end portion, a flex circuit attached or adjacent to the clip body, and an elastomeric sleeve that envelops at least part of the first end portion and at least part of the flex circuit attached or adjacent thereto, or at least part of the second end portion and at least part of the flex circuit attached or adjacent thereto.

US 2008/302364 A1 discloses a therapy system adapted to treat a patient's ventilatory instability using a ventilatory therapy, a gas modulation therapy, or both. The algorithm implemented by the therapy system monitors the ventilatory instability, such as Cheyne Stokes Respiration, mixed apneas, CPAP emergent apneas, and complex sleep disordered breathing and treats the ventilatory instability. The algorithm also determine a reference point with respect to the ventilatory instability. The therapy delivery system initiates the treatment based on the reference point.

There is a critical unmet need in the field of medicine for non-invasive measurement of respiratory parameters in spontaneously breathing patients. Presently, most respiratory monitoring equipment is used for patients receiving mechanical ventilation. Because most mechanically ventilated patients are intubated, many respiratory parameters can be precisely measured in a way not possible with non-intubated patients. Such parameters include those obtained from capnometry, (end tidal C02 [EtC02], respiratory rate and C02 waveform measurements) and those obtained from respiratory monitors such as differential pressure transducers, absolute pressure transducers and flow transducers (tidal volume [VT], airway pressure [Paw], minute ventilation [VE], respiratory rate [RR], respiratory effort/work of breathing [RE/WOB], inspiratory: expiratory ratio [I:E] and deadspace measurements).

Thus, while patients in the OR and ICU may receive intensive respiratory monitoring, similarly reliable monitoring is not presently available for non-intubated patients who are often ambulatory, such as those on general care floors and other areas of the hospital. Numerous organizations, including the U.S. Food and Drug Administration, the American Society of Anesthesiologists, and the Anesthesia Patient Safety Foundation, have noted this lack of monitoring to be problematic and are calling for new technological advances to migrate intensive respiratory monitoring to non-intubated patients. There is also a critical need for improved monitoring of patients receiving patient controlled anesthesia (PCA) since some central nervous system depressants such as opioids may lead to respiratory depression and subsequent morbidity or mortality. Efforts to preemptively identify patients likely to suffer respiratory depression or respiratory arrest have been only partially successful and adequate monitoring solutions are still lacking even if such patients are identified.

### SUMMARY OF EMBODIMENTS OF THE INVENTION

The invention is a system according to claim 1.

### BRIEF DESCRIPTION OF THE DRAWINGS

The following drawings are provided to illustrate various aspects related to the present inventive concept and are not intended to limit the scope of the present invention.
FIGURE 1A and IB illustrate a zero cross method and a band cross method, respectively, for determining whether a respiratory effort occurred based on the PPG signal.

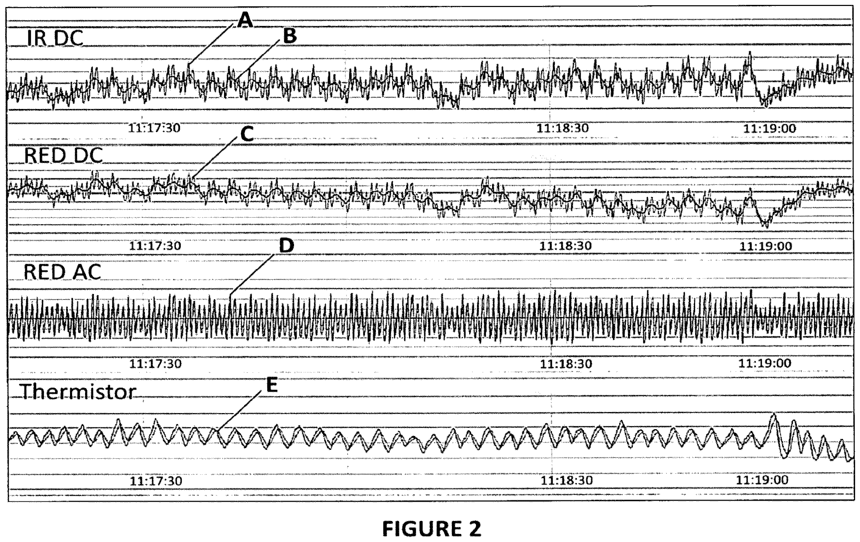

**FIGURE 2** provides the DC component signal stream obtained from an infrared light emitter (B) and red light emitter (C), along with a red AC component signal stream (D) and a thermistor signal stream (E). The AC and DC component signal streams are obtained from the raw PPG signal (A).
**FIGURE 3** provides a table providing likely physiological causes for particular combinations of PPG and thermistor signal readings.

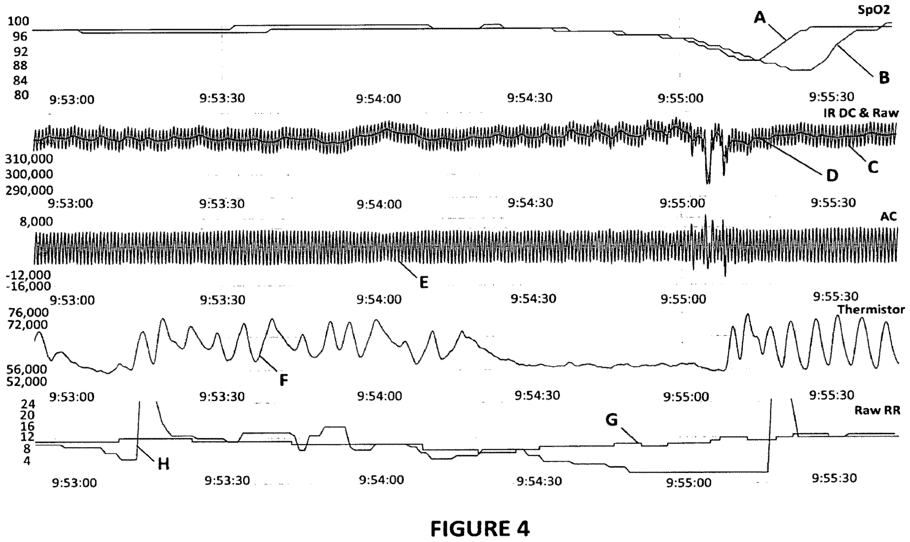

**FIGURE 4** provides PPG, thermistor and capnometry data over time for a patient in the operating room who is having periods of obstructive apnea typical of obstructive sleep apnea. Signals "A" and "B" show the blood oxygen saturation (SpO2) over time obtained from a patient's nasal alar and finger, respectively. The signal "C" is the raw PPG signal, while signal "D" is the processed DC component of the PPG signal. Signal "E" is the AC component signal. Signal "F" is the thermistor signal (nasal air flow) over time. Signal "G" is the respiration rate over time as determined by the processed AC component of the PPG signal and signal "H" is the capnometry respiratory rate over time.
**FIGURE 5** provides PPG and thermistor data over time for a patient in the operating room who is having periods of obstructive apnea typical of obstructive sleep apnea. Signals "A" and "B" show the blood oxygen saturation (SpO₂) over time obtained from a patient's nasal alar and finger, respectively. The signal "C" is the raw PPG signal, while signal "D" is the DC component of the PPG signal. Signal "E" is the AC component signal. Signal "F" is the thermistor signal (nasal air flow) over time.

### DETAILED DESCRIPTION OF EMBODIMENTS OF THE INVENTION

Various embodiments of the disclosure will be described more fully hereinafter with reference to the accompanying drawings, in which embodiments of the disclosure useful to understand the present invention are shown. The scope of the invention is defined by the appended claims and does not cover the methods disclosed in the present description.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

It will be understood that when an element is referred to as being "on" or "adjacent" to another element, it can be directly on or directly adjacent to the other element or intervening elements may also be present. In contrast, when an element is referred to as being "directly on" or "directly adjacent" to another element, there are no intervening elements present. It will also be understood that when an element is referred to as being "connected" or "coupled" to another element, it can be directly connected or coupled to the other element or intervening elements may be present. In contrast, when an element is referred to as being "directly connected" or "directly coupled" to another element, there are no intervening elements present. Like numbers refer to like elements throughout the specification.

It will be understood that, although the terms first, second, etc. may be used herein to describe various elements, these elements should not be limited by these terms. These terms are only used to distinguish one element from another. Thus, a first element discussed below could be termed a second element without departing from the teachings of the present invention.

Provided according to embodiments of the present invention are methods and systems for monitoring the effectiveness of respiratory attempts in individuals. Methods and systems described herein compare the data output from at least one photoplethysmography (PPG) sensor and at least one secondary respiration sensor to determine whether one or more respiratory attempts occurred and whether the attempt(s) resulted in effective ventilation (exchange of oxygen and carbon dioxide). The comparison of the data from the PPG sensor(s) and the secondary respiration sensor(s) may be used to assess whether the individual is suffering from respiratory distress, for example, is apneic (central or obstructive), hypopneic or hyperpneic. The data from multiple sensors may also be used to better correlate the generated signals with the actual physiological processes occurring in the individual.

### Definitions

As used herein, an individual, also referred to as a patient, includes any mammal, including humans of any age. The individual may be monitored in any care setting including, but not limited to, hospitals *(e.g.,* operating room (OR), intensive care unit (1CU),general care floors, or during transport therein); nursing homes, medical offices, medical transport and homes.

As used herein, a "raw PPG signal" includes both completely unprocessed signals and those that have been conditioned. In some cases, the raw PPG signals are "conditioned" or filtered before the signal processing methods described herein. In general, such conditioning is achieved by band pass filters, which may filter out undesirably high or low frequency noise in the signal.

As used herein, the term "respiratory attempt" is meant to refer to an attempt by the individual to take a breath, whether or not ventilation occurs. Respiratory attempts imply that the muscles of respiration are contracting in response to signals from the brainstem. The degree of contraction of the respiratory muscles determines the tidal volume (V_{T}) when the airway is patent. If airway obstruction occurs, muscle contraction may result in decreased V_{T} or in the case of complete obstruction, no airway movement despite muscle contraction. Numerous brainstem inputs including the arterial oxygen saturation (PₐO₂), arterial CO₂ (PₐCO₂) and inputs from various receptors in the respiratory muscles determine the degree of contraction of the respiratory muscles. Disease states (CNS and non-CNS), medications (e.g. opioids, benzodiazepines, etc) and other inputs may alter the "gain" of the brainstem and may decrease or prevent contraction of the respiratory muscles.

As used herein, the term "respiratory air flow/volume" refers to the gas flow/volume in and out of the airways and lungs of an individual, and includes both air flow in the conducting airways (dead space) in the individual's respiratory system and to actual ventilation.

As used herein, "ventilation" is meant to refer to air movement that results in exchange of CO₂ and oxygen in the individual. A "ventilation" may also be referred to herein as a "breath".

### Photoplethysmography (PPG)

PPG is commonly used for the determination of blood oxygen saturation (SₚO₂). This is termed pulse oximetry and is based on the absorption characteristics of red and IR light at different hemoglobin saturations. Because the path length of the light is not fixed, the "ratio of ratios" of the AC and DC components of the two wavelengths is used to calculate SₚO₂. In "classic" oxygen saturation measurements, it is assumed that the important information is contained in the "AC" component of the PPG signal and that the DC is only used to determine the "offset" of the signal from the baseline. The inventors have determined that both the AC and DC components of the PPG signals obtained at or on the head (including at the nose or ears), and in particular, at the nasal alae or other sites at or near the nose (*e.g.,* columella, nasal septum, over the ophthalmic artery) contain valuable respiratory information that is unavailable from conventional digit based PPG measurements.

Thus, while in some embodiments of the invention, the raw signals are used to determine the respiratory parameters described herein, in some embodiments of the invention, the PPG signals are separated into AC and/or DC component signal streams, and the isolated AC and/or DC signal stream may be used to monitor respiration in the individual. In particular embodiments, the DC component stream is used to monitor respiration. The separation of the AC and DC component signal streams may be achieved by a number of different methods, but in some embodiments, the components are separated as discussed in U.S. Patent No. 8,529,459, which is herein incorporated by reference in its entirety. As another example, in some embodiments, the DC component signal stream is determined by interpolating the peaks of the raw signal stream and interpolating the troughs of the combined signal stream and then averaging the two interpolated lines (interpolated peak line and interpolated trough line) to form the DC component signal stream. Other methods of separating AC and DC components of PPG signals that are known in the art may also be used in some embodiments.

While in some embodiments, only one of the raw, AC and DC component signal streams is monitored to analyze respiration in the individual, in some embodiments, more than one of the raw, AC and DC component signal streams are monitored. One reason for monitoring both the AC and DC component signal streams is that the AC and DC components both may provide information regarding respiration (including respiratory rate, effort, obstruction, and the like) and the strength of each signal may vary based on the position or physiological condition of the individual.

The amplitude of the PPG waveforms (AC, DC and raw) may vary with changes in blood volume reflecting the effects of intrathoracic pressure changes throughout the respiratory cycle on differential volume in the right and left ventricle and therefore the carotid arteries. The changes in the DC (and to a lesser degree that AC) component show the inspiration and expiration of a respiratory effort and thus, a presumed respiration rate (RR) can be calculated based on the respiratory efforts. Body position and the degree of airway obstructions (AO) also affect the amplitude of the AC and DC component signals. For instance, because more blood is present in the head when patients are reclining (prone or supine) or in a head down position, the PPG signal (amplitude) is generally larger and the respiratory efforts are easier to identify. Increased tidal volume and airway obstruction may also lead to larger amplitude of the PPG signals due to the increase in intrathoracic pressure.

The PPG signals may vary with the intrathoracic pressure from an attempted breath, and thus, these PPG signals can be used to measure the frequency of respiratory attempts, and the relative effort associated with each attempt. In the absence of obstruction or ineffective ventilation (only deadspace), the rate of respiratory attempts may correlate with the individual's respiration rate. However, if there is complete airway obstruction (as with obstructive sleep apnea) or ineffective ventilation (only deadspace), there may be more respiratory attempts than effective breaths (ventilation). As such, the number of respiratory attempts is always equal to or greater than the number of breaths.

Therefore, in some embodiments of the present invention, the raw, AC and/or DC component signal streams may be evaluated to determine whether the individual has made a respiratory attempt. In particular embodiments, the DC component signal stream is evaluated to determine if a respiratory effort has occurred. In some embodiments, to make this determination, a computer may evaluate the amplitude of the PPG signal stream over time. As an increase in intrathoracic pressure will cause the amplitude of the PPG signal to increase, upon inhalation the amplitude of the PPG signal should increase. Likewise, upon exhalation, the intrathoracic pressure decreases and the PPG signal decreases. Thus, peaks are generated in the PPG signal when effective or ineffective respiratory efforts occur. Criteria can be set for determining whether an increase and decrease in amplitude is sufficiently large to indicate that a respiratory attempt has occurred. In some cases, the area under the curve or area above the curve, or other similar waveform parameters for a plethysmography waveform may be used, alone or in combination with the amplitude, to determine whether a respiratory attempt has occurred.

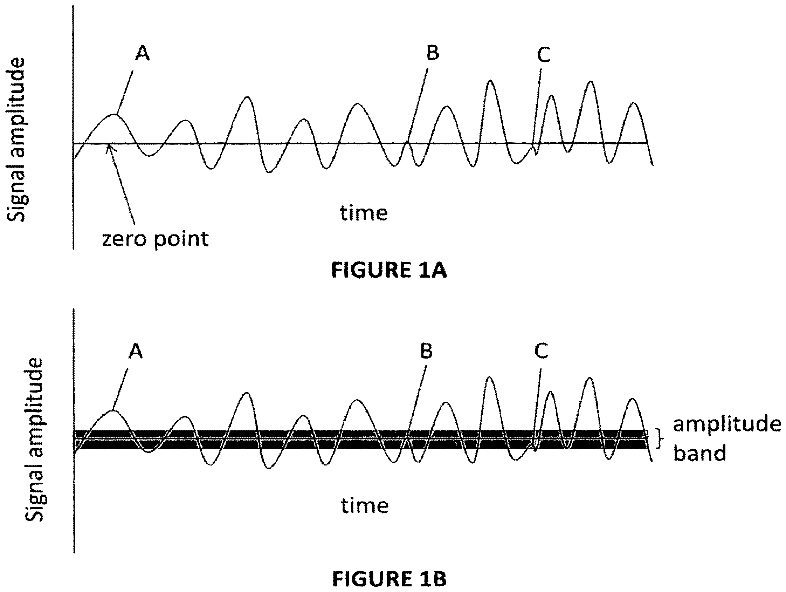

Referring to **Figure 1A****,** a PPG signal stream (e.g., a DC component signal stream) may be evaluated over time. In some embodiments, as the amplitude of the signal over time changes to suggest that a breath has occurred (e.g., increase in amplitude for inspiration followed by a decrease for expiration), a zero cross method may be used to determine whether a respiratory attempt has occurred. In such methods, a zero point (baseline) may be determined and if the PPG signal stream amplitude crosses the zero point twice (once for inspiration and once for expiration), a breath is deemed to have been attempted. Peaks "A" and "B" cross the zero point twice and so are considered to be a respiratory attempts, while peak "C" does not cross the zero point line and so is not considered a respiratory attempt. In some embodiments, the zero point is the average amplitude over some predetermined time range, such as over the previous 1, 2, 5 or 10 minutes. In some cases, once the signal crosses the zero point, the computer may determine whether the signal crossing is statistically valid (e.g., via a t-test) and if not, the process may then be iterated.

Referring to **Figure 1B****,** in some embodiments, a "band cross" method may be used such that an amplitude range centered around a zero crossing point may be assigned to the signal, and the size of the band may be determined, for example as fixed amplitude band (e.g., average amplitude +/- a predetermined range) or as a percentage of the total signal amplitude, and in some cases may be determined over a predetermined time range, such as over the previous 1, 2, 5 or 10 minutes. In such cases, a respiratory effort is determined to have occurred when the signal crosses the amplitude band twice (inspiratory effort and expiratory effort). This is analogous to the zero point crossing method but instead of a single point, the signal must cross the amplitude band for a respiratory effort to have been deemed to occur. This decreases the likelihood that noise in the signal will affect the determination of respiratory efforts. In some embodiments, once the signal crosses the amplitude band, the computer may determine whether the signal crossing is statistically valid (*e.g.,* via a t-test) and if not, the process may then be iterated. In **Figure 1B****,** Peak "A" is deemed to be a respiratory effort, while Peaks "B" and "C" would not be sufficiently large to be deemed respiratory efforts by the band cross method.

In some cases, either alone or in combination with the zero or band cross methods, the magnitude of the increase in signal over the baseline may be used to assess the degree of respiratory effort, which may provide information regarding the presence and degree of obstruction (*i.e.,* a large and/or increasing amplitude may suggest obstruction).

While in general, the methods described herein relate to PPG signals from a central site on or at the head, if a respiratory attempt could be determined from other PPG sensors at the digits or elsewhere on the body, then the methods and systems described herein may be used with PPG signals obtained at any location on the body.

### Secondary Respiration Sensors

Secondary respiration sensors are used to compare with the respiratory information obtained from the PPG sensor(s). Such sensors include, but are not limited to, nasal air flow sensors, nasal pressure sensors, capnometers, thermistors, acoustic sensors, differential pressure transducers, chest or abdominal bands, and the like. In some cases, both the PPG sensor(s) and the secondary respiration sensor(s) are situated at the nose, and in some cases, a single device or system (e.g., an array) may include both the PPG sensor(s) and the secondary respiration sensor(s).

In some embodiments, the secondary respiration sensor may detect respiratory airflow or temperature changes at the nostril, such as with a thermistor. For example, during inspiration, a thermistor placed at the nostril detects a relative decrease in temperature compared to exhalation since, in most situations, body temperature, and therefore exhaled breath temperature, is higher than ambient temperature. Thus, detection of changes in temperature may be a suitable means to determine respiratory air flow and therefore, respiratory rate. Air flow from one or both nostrils may be monitored and compared with the PPG information.

As another example, capnometry may provide a number of respiratory parameters. Such parameters may generally be reliably used for monitoring adequacy of ventilation if the patient is intubated. Unfortunately both hyper- and hypoventilation in patients may cause the results to be unreliable. However, in some cases, capnometry may be useful as a secondary respiration sensor to detect the respiratory airflow and thus, may be helpful to determine whether respiratory attempts lead to effective ventilation.

The respiratory data from the secondary respiration sensor may be handled analogously to the PPG data. As the amplitudes of the secondary respiration sensor signals may change with respiratory air flow during inhalation and exhalation, a zero cross method may be used to measure whether respiratory airflow is sufficient to be deemed ventilation. In such methods, a zero point (baseline) may be determined and if the secondary respiration sensor (*e.g.,* thermistor) signal stream amplitude crosses the zero point twice (once for inspiration and once for expiration), ventilation is deemed to have occurred. If the waveforms in **Figures 1A** were thermistor waveforms instead of PPG waveforms, Peaks "A" and "B" cross the zero point twice and so would be considered to be sufficient to result in ventilation, while peak "C" does not cross the zero point line and so would not be considered sufficiently large to indicate ventilation. In some embodiments, the zero point is the average amplitude over some predetermined time range, such as over the previous 1, 2, 5 or 10 minutes. In some cases, once the signal crosses the zero point, the computer may determine whether the signal crossing is statistically valid (*e.g.,* via a t-test) and if not, the process may then be iterated.

In some embodiments, a "band cross" method may be used such that an amplitude range centered around a zero crossing point may be assigned to the signal, and the size of the band may be determined, for example as fixed amplitude band (e.g., average amplitude +/- a predetermined range) or as a percentage of the total signal amplitude, and in some cases may be determined over a predetermined time range, such as over the previous 1, 2, 5 or 10 minutes. In such cases, ventilation is determined to have occurred when the signal crosses the amplitude band twice (inspiratory effort and expiratory effort). In some embodiments, once the signal crosses the amplitude band, the computer may determine whether the signal crossing is statistically valid *(e.g.,* via a t-test) and if not, the process may then be iterated. In the waveform in **Figure 1B** was a thermistor waveform instead of a PPG waveform, Peak "A" would be deemed to indicate ventilation, while Peaks "B" and "C" would not be sufficiently large to be deemed ventilation by the band cross method.

In some cases, either alone or in combination with the zero or band cross methods, the magnitude of the increase or decrease in the signal relative to a baseline value may be used to assess the depth of breathing, which may provide information regarding whether hypopnea, apnea or hyperpnea is occurring. For example, if the amplitude of the thermistor or other secondary respiration sensor becomes "high" (*e.g.,* twice a baseline amplitude or greater), then hyperpnea may be indicated. If the amplitude of the thermistor or other secondary respiration sensor becomes "low" (e.g., 25% or less of a baseline amplitude), then hypopnea and or lack of ventilation may be deemed to have occurred.

### Methods of Respiratory Monitoring using PPG and Secondary Respiration Sensors

As described above, a computer may evaluate the PPG data and the secondary respiration sensor(s) data to identify whether there has been an attempted breath (PPG) and whether the respiratory attempt resulted in ventilation (secondary respiration sensor). The combination of the two data streams may also be used to identify physiological processes and problems and to track the respiratory function of the individual.

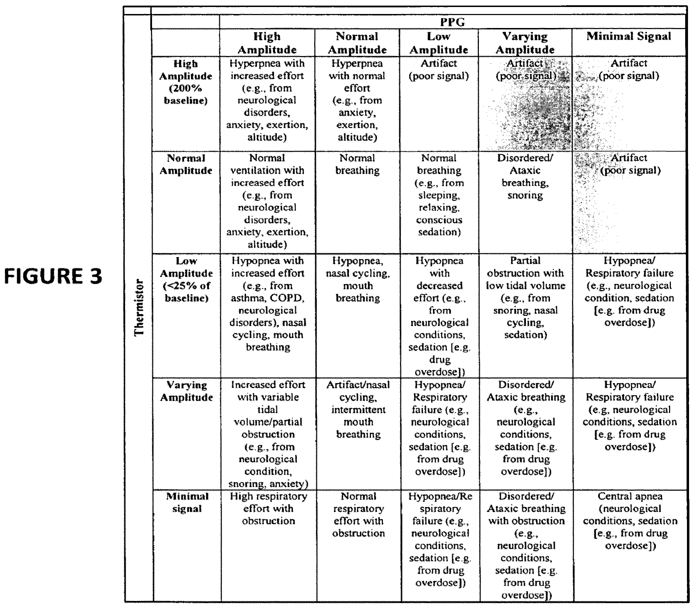

**Figure 3** provides a list of respiratory processes or problems (and their possible causes), along with how they might be diagnosed by analysis of the PPG and secondary respiration sensor (e.g., a thermistor) signals. In some embodiments, a predetermined reaction may be effected based on which physiological process or problem is indicated by the two signals. For example, medication may be administered (*e.g.,* anti-anxiety agent, narcotic reversal agent, anti-asthma medication, and the like), oxygen may be administered, the patient may be alerted, or medical staff may be alerted.

The ability to distinguish respiratory attempts from effective ventilation may be extremely useful in a clinical setting, both to identify patients in respiratory distress and to decrease false alarms that may be present with other sensors such as thermistors or capnomters (when used by themselves). While this analysis may be on a breath-to-breath basis, analysis of the trend or pattern of breathing over a particular time period may be clinically useful. For example, the total number of respiratory attempts vs. the number of effective breaths for a given time period may be useful to assess the ventilation status of the patient. The percentage of ventilations vs. the total respiratory attempts may be measured at particular time intervals. The trend in this parameter over time may indicate an increase or decrease in the individual's respiratory function. Thus, a predetermined reaction (*e.g.,* alarm, medication or oxygen administration, alerting of individual or staff, etc.) may be effected if the percentage decreases below a certain value. In some cases, a predetermined reaction (*e.g.,* increase or decrease in medication or oxygen) may be effected if the percentage of effective ventilations increases above a certain value.

As a particular example, a patient may be breathing spontaneously with adequate tidal volume to have effective gas exchange. At a later point in time, medication may be administered and/or the patient may fall asleep. At such time, the PPG amplitude and the area under the curve (AUC) may fall while a thermistor shows smaller breaths. The combination of a decrease in the PPG amplitude and the thermistor amplitude are indicative of hypopnea and hypoventilation.

Further, the use of PPG data plus the secondary respiration data can allow for the characterization of obstructive apnea from central apnea. With obstructive apnea, the PPG demonstrates continued and/or increasing respiratory efforts, but the secondary respiration sensor shows less or now air flow indicating the lack of effective ventilation. With central apnea, the PPG signal does not show respiratory attempts and the secondary respiration sensor shows no ventilation in the patient.

Blood oxygen saturation measurements may be used to confirm this determination and may be particulary helpful in defining when an alarm is effected. Research by the inventors has shown that sensors at the nose respond more quickly to decreases in oxygen saturation than sensors placed on digits Thus, sensors on the nose provide an "early warning" of oxygen desaturation (from hypoventilation, atalectasis, or airway obstruction, for example). In the absence of desaturation, changes detected by the PPG and thermistor (or other respiratory sensors) can be trended, thus reducing the number of false alarms, but still providing feedback to healthcare providers that the patient's condition is deteriorating, albeit, at a slower rate. Trend analysis can be particularly valuable in pinpointing the cause of changes in respiration, and in reducing false alarms while providing pertinent information presently unavailable to the healthcare team.

In the present invention, respiration is monitored by comparing the respiration rate from the PPG (e.*g*, respiratory efforts/min) to respiration rate (*e.g.,* ventilations/min) derived from the secondary respiration sensor. When these rates agree (within a predetermined tolerance), the respiratory attempts are considered successful (ventilation occurs). When these rates diverge such as when the secondary respiration sensor RR decreases by a certain percentage (e.g., 10, 20, 30% or more), then the patient may be considered apneic or hypopneic to the point of hypoventilation or apnea. In some cases, if this divergence lasts longer than a predetermined time period, e.g., 20-30 seconds (the length of time for defined apnea), and in some cases, the saturation has declined (for instance greater than 3%), an alarm is generated (or other predetermined reaction discussed above is effected). In some cases, if the divergence in PPG respiration rate diverges from the respiration rate from the secondary respiration sensor for the predetermined amount of time, but desaturation does not occur, a second predetermined reaction may be generated (e.g., an error message may be generated, additional data is obtained, etc.).

One or more algorithms may be used to combine or fuse the PPG data with the data from the secondary respiration sensor. This algorithm may use multivariate analysis to filter and demodulate physiological signals from the sensors to provide information on the desired parameters. The current quality and trending quality of the input signals may also be measured. One measure of signal quality is the signal to noise ratio (SNR). By monitoring the SNR and establishing a trend of "normal" signal amplitude, an alarm can be set if the SNR ratio decreases below a threshold determined to reflect the point of adequate ventilation.

In some embodiments of the invention, the PPG sensors may be calibrated to obtain more quantitative information regarding the respiratory effort in the individual. A semi-quantitative respiratory effort (work of breathing) can be calculated by calibrating the PPG sensor(s) at the time of placement and periodically thereafter. A surrogate for respiratory effort can be calibrated by having the patient breath through a number of tubes of known resistance (for instance at resistances of 2cm H₂O/L/sec, 10 cm H₂O/L/sec and 25 cm H₂O/L/sec) at a fixed flow rate (e.g., 15 L/minute) to simulate normal effort, moderately increased effort and markedly increased effort. The patient would inhale through the tubes at a fixed flow rate which, for example, could be easily presented to the patient by placing a reed inside the tube which would provide auditory feedback when the appropriate flow rate is reached. While the patient breaths through the tubes, the PPG amplitude, AUC and AC and DC derived parameters would be measured.

Tidal volume could be estimated from a thermistor (or similar nasal air flow, pressure or volume measuring devices) by having the patient inhale a fixed volume of gas using a face mask connected to a bag containing various volumes of air (e.g. 0.25L, 0.5L, 1.0L and 2.0L). The amplitude and more accurately the AUC of the thermistor signal would be proportional to the tidal volume. Once the system is calibrated, only the PPG and thermistor signals would be needed to continuously monitor the respiratory status of the patient. Calibration could be repeated at regular intervals thereafter.

### Systems for Monitoring Respiration

The methods described herein may be performed by any suitable device, such as, for example, a general-purpose microprocessor (which may include one, two or more individual microprocessors). Such a microprocessor may be adapted to execute software, which may include an operating system and one or more applications, as part of performing the functions described herein. In electronic communication with the microprocessor may be a computer memory, such as a read-only memory (ROM), random access memory (RAM), and the like. Any suitable computer-readable media may be used in the system for data storage. Computer-readable media are capable of storing information that can be interpreted by microprocessor. This information may be data or may take the form of computer-executable instructions, such as software applications, that cause the microprocessor to perform certain functions and/or computer-implemented methods. Depending on the embodiment, such computer-readable media may include computer storage media and communication media.

Computer storage media may include volatile and non-volatile, removable and non-removable media implemented in any method or technology for storage of information such as computer-readable instructions, data structures, program modules or other data. Computer storage media may include, but is not limited to, RAM, ROM, EPROM, EEPROM, flash memory or other solid state memory technology, CD-ROM, DVD, or other optical storage, magnetic cassettes, magnetic tape, magnetic disk storage or other magnetic storage devices, or any other medium which can be used to store the desired information and which can be accessed by components of the system.

In particular embodiments, a microprocessor may determine whether a respiratory attempt has occurred based on the signals from the PPG sensor and whether this attempt is successful based on the signals from the secondary respiration sensor. In particular, the microprocessor may isolate the AC and DC components of the PPG signal (*e.g.,* as discussed *supra* and by algorithms that may be stored in the computer storage media in electrical communication with the microprocessor). Such AC and DC components may be analyzed (e.g., as discussed *supra*) to assess whether a respiratory attempt has occurred. The microprocessor may also receive the signal from the secondary respiration detector and process and analyze such signals (e.g., as discussed *supra* and by algorithms that may be stored in the computer storage media in electrical communication with the microprocessor) to identify whether respiratory air flow occurred, and whether it was sufficiently large to be deemed ventilation.

The microprocessor may also then compare the determination of whether the respiratory attempt has occurred (optionally in combination with the confidence level of the determination). If a respiratory attempt is deemed to have occurred by the PPG sensor and the respiratory airflow detected by the secondary respiration detector is sufficient to be deemed ventilation, in some embodiments, a breath will be deemed to have occurred and the microprocessor will include this data in any monitor function or physiological parameter calculation (e.g., respiration rate). If no respiratory attempt and no ventilation occurs, then no breath is counted. In some cases, this may indicate central apnea and if no breathing occurs for a predetermined amount of time (e.g., for a time in a range of 10 to 20, 30 or 40 seconds), an alarm may sound, oxygen administered or other action may be taken to promote ventilation and/or oxygenation of the individual. For example, the patient may be alerted or stimulated, such as via a wisp of air on the cheek, vibration of a monitor or other devices and methods of rousing the individual from sleep or a respiratory depressed state. The respiration rate may also be calculated for each sensor separately and the respiration rates compared, as discussed *supra,* whereby a predefined divergence between the two respiration rates may result in a predetermined reaction, such as an alarm, oxygen administration (or as discussed above).

### Example 1

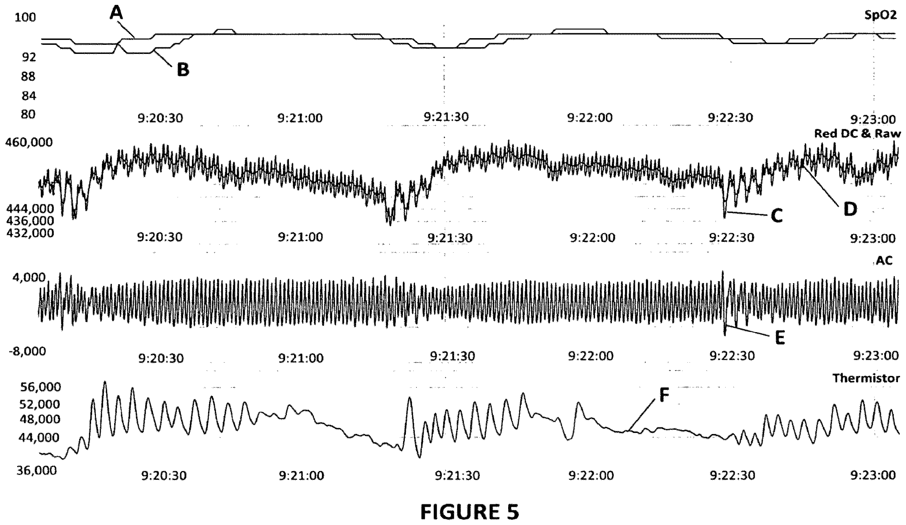

**Figures 4** and **5** show the PPG and thermistor signal streams for a patient in the operating room who is having periods of obstructive apnea typical of obstructive sleep apnea. The combination of the PPG data with the thermistor gives a reliable picture of respiratory status of the patient. The PPG provides oxygen saturation over time ("SpO2"), as well as IR DC and Raw and AC waveforms. The thermistor indicates the air flow from the nostrils over time. In this case, the thermistor provides the most reliable data during periods of a patent airway and adequate tidal volume, and the PPG data is more reliable when the patient is partially or completely obstructed (although the signal may work well most of the time and is not affected by obstruction or preferential nasal flow as may be the case with the thermistor) and amplitude is a indicates "effort".

Referring to **Figure 4****,** waveform "A" denotes the oxygen saturation obtained at the nasal alar, while the "B" waveform denotes oxygen saturation obtained at the finger. It is noted that the desaturation is detected at the nasal alar several seconds before it is detected at the finger. The "C" waveform is the raw PPG signal, while the "D" waveform is the IR DC component signal. The "E" waveform is the AC component signal stream. The "F" waveform is the thermistor waveform. It is noted that when the thermistor loses signal, the IR DC signal becomes more pronounced, the individual increases respiratory efforts against an apparent obstruction. An "arousal" similar after the period of apnea may also be seen in both the PPG signal and where the thermistor signal shows a return to ventilation. Thus, PPG plus thermistor plus oximetry from the nasal ala allows a comprehensive picture of the respiratory status of the patient. As shown in **Figure 4****,** data from a capnometer (waveform "H", which generates raw respiration rates shown in waveform "G") may also be used with PPG either in combination with or in lieu of the thermistor. Waveforms A-F in **Figure 5** are the same as those identified with respect to **Figure 4****.**

## Claims

1. A system for monitoring the effectiveness of respiration in an individual, the system comprising a microprocessor,
wherein the microprocessor is adapted to receive photoplethysmography (PPG) signals obtained from a PPG sensor secured to the individual and signals from a secondary respiration sensor,
wherein the microprocessor is configured to:
determine whether a respiratory attempt in the individual occurred within a predefined time period based on the PPG signals;
determine whether a ventilation occurred within the predefined time period using the signals obtained from the secondary respiration sensor; and
compare the determination of the respiratory attempts based on the PPG signals with the determination of the ventilation based on the secondary respiration sensor to assess whether the individual's respiratory attempts are effective,
wherein the system is **characterized in that** the microprocessor is further configured to calculate a first respiration rate based on the respiratory attempts determined from the PPG signals, calculating a second respiration rate based on breaths determined from the secondary respiration sensor, and comparing the first and second respiration rates to determine whether the individual is suffering from hyperpnea, apnea or hypopnea.

2. The system of claim 1, wherein the system comprises a secondary respiration sensor comprising at least one of a thermistor, capnometer, nasal flow sensor, differential pressure transducer, and a nasal pressure sensor.

3. The system of claim 1, wherein if a respiratory attempt is determined to have occurred based on the PPG signals and ventilation is determined to have occurred based on the secondary respiration sensor signals, then the effective respiratory attempt is used to calculate the respiration rate of the individual.

4. The system of claim 1, wherein if a respiratory attempt is determined to have occurred but ventilation is determined to have not occurred, then a predetermined action is effected by the microprocessor.

5. The system of claim 4, wherein the predetermined reaction comprises initiating an alarm signal.

6. The system of claim 4, wherein the microprocessor is configured to calculate the individual's blood oxygen saturation and to determine whether the individual's blood oxygen saturation has decreased to a predefined level, and if so, to effect a predetermined reaction.

7. The system of claim 1, wherein if the individual is determined to be suffering from hyperpnea, apnea or hypopnea, a predetermined reaction is effected by the microprocessor.

8. The system of claim 7, wherein the predetermined reaction comprises initiating an alarm signal.

9. The system of any of claims 1-8, wherein the microprocessor is configured to determine at least one of (a) whether ventilation has occurred, (b) whether the patient has central apnea, and (c) whether the patient has obstructive apnea.

10. The system of claim 9, wherein the microprocessor is further configured to isolate an AC and a DC component signal of the PPG signals, use the isolated AC and/or DC component signal to determine whether a respiratory attempt in the individual has occurred.

11. The system of claim 1, wherein a predetermined reaction is effected by the microprocessor if the respiratory attempt is determined to not have been effective.

12. The system of claim 11, wherein the predetermined reaction comprises initiating an alarm signal.

13. The system of claim 11, wherein the microprocessor is configured to calculate the individual's blood oxygen saturation and to determine whether the individual's blood oxygen saturation has decreased a predefined percentage, and the predetermined reaction is effected if the respiratory attempt is determined to not have been effective and the blood oxygen saturation has decreased a predefined percentage.

## Patentansprüche

1. System zum Überwachen der Wirksamkeit einer Atmung bei einem Individuum, wobei das System einen Mikroprozessor umfasst,
wobei der Mikroprozessor dazu angepasst ist, Photoplethysmographie- (PPG-) Signale, die von einem an dem Individuum befestigten PPG-Sensor erhalten werden, sowie Signale von einem sekundären Atmungssensor zu empfangen,
wobei der Mikroprozessor konfiguriert ist zum:
Bestimmen, ob ein Atemversuch bei dem Individuum innerhalb eines vordefinierten Zeitraums stattgefunden hat, basierend auf den PPG-Signalen;
Bestimmen, ob innerhalb des vordefinierten Zeitraums eine Beatmung stattgefunden hat, unter Verwendung der Signale, die von dem sekundären Atmungssensor erhalten werden; und
Vergleichen der Bestimmung der Atemversuche basierend auf den PPG-Signalen mit der Bestimmung der Beatmung basierend auf dem sekundären Atmungssensor, um zu beurteilen, ob die Atemversuche des Individuums wirksam sind,
wobei das System **dadurch gekennzeichnet ist, dass** der Mikroprozessor weiter dazu konfiguriert ist, eine erste Atemfrequenz basierend auf den von den PPG-Signalen bestimmten Atemversuchen zu berechnen, eine zweite Atemfrequenz basierend auf den vom sekundären Atmungssensor bestimmten Atemzügen zu berechnen, und die erste und die zweite Atemfrequenz zu vergleichen, um zu bestimmen, ob das Individuum an Hyperpnoe, Apnoe oder Hypopnoe leidet.

2. System nach Anspruch 1, wobei das System einen sekundären Atmungssensor umfasst, der mindestens einen von einem Thermistor, Kapnometer, Nasenflusssensor, Differenzdruckwandler und Nasendrucksensor umfasst.

3. System nach Anspruch 1, wobei, wenn basierend auf den PPG-Signalen bestimmt wird, dass ein Atemversuch stattgefunden hat, und basierend auf den sekundären Atmungssensorsignalen bestimmt wird, dass eine Beatmung stattgefunden hat, der effektive Atemversuch dazu verwendet wird, die Atemfrequenz des Individuums zu berechnen.

4. System nach Anspruch 1, wobei, wenn bestimmt wird, dass ein Atemversuch stattgefunden hat, jedoch bestimmt wird, dass keine Beatmung stattgefunden hat, eine vorbestimmte Aktion von dem Mikroprozessor veranlasst wird.

5. System nach Anspruch 4, wobei die vorbestimmte Reaktion Auslösen eines Alarmsignals umfasst.

6. System nach Anspruch 4, wobei der Mikroprozessor dazu konfiguriert ist, die Sauerstoffsättigung im Blut des Individuums zu berechnen und zu bestimmen, ob die Sauerstoffsättigung im Blut des Individuums auf ein vordefiniertes Niveau gesunken ist, und, falls dies der Fall ist, eine vorbestimmte Reaktion zu veranlassen.

7. System nach Anspruch 1, wobei eine vorbestimmte Reaktion von dem Mikroprozessor veranlasst wird, wenn bestimmt wird, dass das Individuum an Hyperpnoe, Apnoe oder Hypopnoe leidet.

8. System nach Anspruch 7, wobei die vorbestimmte Reaktion Auslösen eines Alarmsignals umfasst.

9. System nach einem der Ansprüche 1-8, wobei der Mikroprozessor dazu konfiguriert ist, mindestens eines davon zu bestimmen, (a) ob eine Beatmung stattgefunden hat, (b) ob der Patient zentrale Apnoe aufweist und (c) ob der Patient obstruktive Apnoe aufweist.

10. System nach Anspruch 9, wobei der Mikroprozessor weiter dazu konfiguriert ist, ein WS- und ein GS-Komponentensignal der PPG-Signale zu isolieren, das isolierte WS- und/oder GS-Komponentensignal zu verwenden, um zu bestimmen, ob ein Atemversuch bei dem Individuum stattgefunden hat.

11. System nach Anspruch 1, wobei eine vorbestimmte Reaktion von dem Mikroprozessor veranlasst wird, wenn bestimmt wird, dass der Atemversuch nicht wirksam war.

12. System nach Anspruch 11, wobei die vorgegebene Reaktion Auslösen eines Alarmsignals umfasst.

13. System nach Anspruch 11, wobei der Mikroprozessor dazu konfiguriert ist, die Sauerstoffsättigung im Blut des Individuums zu berechnen und zu bestimmen, ob die Sauerstoffsättigung im Blut des Individuums um einen vordefinierten Prozentsatz gesunken ist, und die vorbestimmte Reaktion veranlasst wird, wenn bestimmt wird, dass der Atemversuch nicht wirksam war und die Sauerstoffsättigung im Blut um einen vordefinierten Prozentsatz gesunken ist.

## Revendications

1. Système pour surveiller l'efficacité de la respiration chez un individu, le système comprenant un microprocesseur,
dans lequel le microprocesseur est conçu pour recevoir des signaux de photopléthysmographie (PPG) obtenus à partir d'un capteur PPG fixé à l'individu et des signaux provenant d'un capteur respiratoire secondaire,
dans lequel le microprocesseur est configuré pour :
déterminer si une tentative respiratoire chez l'individu s'est produite au cours d'une période de temps prédéfinie sur la base des signaux PPG ;
déterminer si une ventilation s'est produite dans la période de temps prédéfinie à l'aide des signaux obtenus à partir du capteur de respiration secondaire ; et
comparer la détermination des tentatives respiratoires basée sur les signaux PPG avec la détermination de la ventilation basée sur le capteur de respiration secondaire pour évaluer si les tentatives respiratoires de l'individu sont efficaces,
dans lequel le système est **caractérisé en ce que** le microprocesseur est en outre configuré pour calculer une première fréquence respiratoire sur la base des tentatives respiratoires déterminées à partir des signaux PPG, calculer une seconde fréquence respiratoire sur la base des respirations déterminées à partir du capteur de respiration secondaire, et comparer les première et seconde fréquences respiratoires pour déterminer si l'individu souffre d'hyperpnée, d'apnée ou d'hypopnée.

2. Système selon la revendication 1, dans lequel le système comprend un capteur de respiration secondaire comprenant au moins l'un parmi une thermistance, un capnomètre, un capteur de débit nasal, un transducteur de pression différentielle et un capteur de pression nasale.

3. Système selon la revendication 1, dans lequel s'il est déterminé qu'une tentative respiratoire s'est produite sur la base des signaux PPG et s'il est déterminé qu'une ventilation s'est produite sur la base des signaux du capteur de respiration secondaire, alors la tentative respiratoire efficace est utilisée pour calculer la fréquence respiratoire de l'individu.

4. Système selon la revendication 1, dans lequel s'il est déterminé qu'une tentative respiratoire s'est produite mais s'il est déterminé qu'une ventilation ne s'est pas produite, alors une action prédéterminée est effectuée par le microprocesseur.

5. Système selon la revendication 4, dans lequel la réaction prédéterminée comprend le déclenchement d'un signal d'alarme.

6. Système selon la revendication 4, dans lequel le microprocesseur est configuré pour calculer la saturation en oxygène du sang de l'individu et pour déterminer si la saturation en oxygène du sang de l'individu a diminué jusqu'à un niveau prédéfini, et si tel est le cas, pour effectuer une réaction prédéterminée.

7. Système selon la revendication 1, dans lequel s'il est déterminé que l'individu souffre d'hyperpnée, d'apnée ou d'hypopnée, une réaction prédéterminée est effectuée par le microprocesseur.

8. Système selon la revendication 7, dans lequel la réaction prédéterminée comprend le déclenchement d'un signal d'alarme.

9. Système selon l'une quelconque des revendications 1-8, dans lequel le microprocesseur est configuré pour déterminer au moins l'un parmi (a) si une ventilation s'est produite, (b) si le patient souffre d'apnée centrale et (c) si le patient souffre d'apnée obstructive.

10. Système selon la revendication 9, dans lequel le microprocesseur est en outre configuré pour isoler un signal de composante alternative et continue des signaux PPG, utiliser le signal de composante alternative et/ou continue isolé pour déterminer si une tentative respiratoire chez l'individu s'est produite.

11. Système selon la revendication 1, dans lequel une réaction prédéterminée est effectuée par le microprocesseur s'il est déterminé que la tentative respiratoire n'a pas été efficace.

12. Système selon la revendication 11, dans lequel la réaction prédéterminée comprend le déclenchement d'un signal d'alarme.

13. Système selon la revendication 11, dans lequel le microprocesseur est configuré pour calculer la saturation en oxygène du sang de l'individu et pour déterminer si la saturation en oxygène du sang de l'individu a diminué d'un pourcentage prédéfini, et la réaction prédéterminée est effectuée s'il est déterminé que la tentative respiratoire n'a pas été efficace et si la saturation en oxygène du sang a diminué d'un pourcentage prédéfini.
